# EUROPÄISCHE PATENTSCHRIFT

(11) **EP 2 397 068 B1**
(45) Veröffentlichungstag und Bekanntmachung des Hinweises auf die Patenterteilung: **27.05.2020**
(21) Anmeldenummer: 11168232.4
(22) Anmeldetag: 31.05.2011
(51) Int. Cl.: A61B 3/16, A61B 3/107

(54) **Analyse des intraokularen Drucks**
Analysis of intraocular pressure
Analyse de la pression intra-oculaire

(30) Priorität: 28.10.2010 DE 102010049634; 28.10.2010 DE 102010049633; 21.06.2010 EP 10166681
(43) Veröffentlichungstag der Anmeldung: 21.12.2011
(73) Patentinhaber: Oculus Optikgeräte GmbH, 35582 Wetzlar-Dutenhofen (DE)
(72) Erfinder: Köst, Gert, 30159 Hannover (DE); Steinmüller, Andreas, 35435 Wettenberg (DE)
(74) Vertreter: advotec.

(56) Entgegenhaltungen:
- EP-A1- 1 692 998
- DE-A1-102006 039 893
- US-A- 6 120 444
- US-A1- 2005 030 473
- US-A1- 2007 121 120
- KEMPF R ET AL: "Understanding eye deformation in non-contact tonometry", CONFERENCE PROCEEDINGS. ANNUAL INTERNATIONAL CONFERENCE OF THE IEEE ENGINEERING IN MEDICINE AND BIOLOGY SOCIETY (IEEE CAT. NO. 06CH37748); 30 AUG.-3 SEPT. 2006; NEW YORK, NY, USA, IEEE, PISCATAWAY, NJ, USA, 30. August 2006 (2006-08-30), Seiten 5428-5431, XP031390671, ISBN: 978-1-4244-0032-4

## Beschreibung

Die Erfindung betrifft ein ophthalmologisches Analyseverfahren zur Messung eines intraokularen Drucks in einem Auge mit einem Analysesystem, sowie ein derartiges Analysesystem, gebildet aus einer Betätigungseinrichtung, mit der eine Hornhaut des Auges berührungsfrei verformt wird, wobei mit der Betätigungseinrichtung ein Luftstoß zur Verformung der Hornhaut auf das Auge aufgebracht wird, einem Beobachtungssystem, mit dem die Verformung der Hornhaut beobachtet und aufgenommen wird, wobei mit dem Beobachtungssystem Schnittbilder der unverformten und verformten Hornhaut aufgenommen werden, und einer Analyseeinrichtung, mit der aus den Schnittbildern der Hornhaut der intraokulare Druck abgeleitet wird, wobei in der Analyseeinrichtung aus den Schnittbildern der Hornhaut eine Materialeigenschaft der Hornhaut abgeleitet wird.

Derartige Analyseverfahren und -systeme sind hinreichend bekannt und dienen primär einer möglichst genauen, berührungslosen Messung eines intraokularen Drucks in einem Auge. Dazu wird beispielsweise ein Non-Kontakt-Tonometer verwandt, mit Hilfe dessen ein Luftstoß auf das zu untersuchende Auge appliziert wird, wobei eine Stärke des Luftstoßes so gewählt wird, dass die Hornhaut des Auges unter Ausbildung einer konkaven Oberflächenform eingedrückt wird. Vor Erreichen eines Maximums einer Verformung der Hornhaut bzw. vor einem Einklappen der Hornhaut in Richtung der Augenlinse, bildet die Hornhaut kurzzeitig eine plane Fläche aus, die als sogenannter erster Applanationspunkt bezeichnet wird. Nach der maximalen Auslenkung der Hornhaut und einem Zurückklappen derselben in den ursprünglichen Zustand, wird ein ebensolcher zweiter Applanationspunkt von der Hornhaut durchlaufen. Durch eine Inbezugsetzung eines Drucks des Luftstoßes mit einem zeitlichen Verlauf der Applanation der Hornhaut ist es nun möglich, einen intraokularen Druck zu ermitteln. Die mit dem Non-Kontakt-Tonometer ermittelten Messwerte werden in Relation zu Vergleichsmesswerten gesetzt, welche mit einem relativ genauer messenden Applanationstonometer bzw. Kontakt-Tonometer ermittelt wurden, so dass als ein Ergebnis ein dem tatsächlichen intraokularen Druck angenäherter Augeninnendruck abgeleitet werden kann.

Jedoch ist ein mit einem Non-Kontakt-Tonometer gemessener intraokularer Druck gegenüber einer Druckmessung mit einem Applanationstonometer noch nicht hinreichend genau, da eine Messung unter anderem von der Hornhaut verfälscht wird. Um eine Messgenauigkeit zu verbessern, wurde daher versucht, den Einfluss der Hornhaut auf die Messung zu berücksichtigen, beispielsweise durch eine Dickenmessung oder eine Messung von Hornhautradien vor Beginn der Non-Kontakt-Tonometermessung. Auch ist es bekannt, einen Elastizitätsmodul bzw. Youngschen-Modul als eine biomechanische Materialeigenschaft der Hornhaut zu berücksichtigen und die betreffende Messung mit einem entsprechenden Berechnungsfaktor zu korrigieren. Dabei wird bei allen Messungen, auch bei unterschiedlichen Augen, davon ausgegangen, dass der Elastizitätsmodul immer gleich groß und somit konstant ist. Weiter wird davon ausgegangen, dass eine Hornhaut einen in allen Bereichen der Hornhaut gleich großen Elastizitätsmodul aufweist. Eine derartige Einbeziehung eines Elastizitätsmoduls in einer Non-Kontakt-Tonometermessung hat den Nachteil, dass diese Materialeigenschaft bzw. dieser Materialkennwert zur Charakterisierung einer Zugbelastung dient, wie Sie bei einer Non-Kontakt-Tonometermessung nicht auftritt. Weiter variiert ein Elastizitätsmodul von Auge zu Auge individuell und auch, in Abhängigkeit von den jeweiligen Hornhautbereichen, innerhalb der Hornhaut selbst. Eine Einbeziehung derartiger Materialkennwerte und eine Berechnung des Messergebnisses können daher noch nicht zu befriedigend genauen Messergebnissen führen.

Weiter ist es bekannt, die biomechanischen Eigenschaften einer Hornhaut während einer Non-Kontakt-Tonometermessung in diese mit einzubeziehen bzw. diese so bereits während der Messung zu ermitteln. Dazu wird ein Luftstoß auf eine Hornhaut aufgebracht, wobei ein Pumpendruck während des Verlaufs der Messung mittels eines Druckaufnehmers kontinuierlich erfasst wird. Weiter wird ein zeitlicher Verlauf der Messung erfasst sowie ein erster und ein zweiter Applanationspunkt der Hornhaut optisch detektiert. Ein intraokularer Druck kann nun beispielsweise durch eine Bestimmung der zum Zeitpunkt der ersten und zweiten Applanation herrschenden Drücke abgeleitet werden, insbesondere da die zur Biegung der Hornhaut notwendigen Kräfte beim Ein- bzw. Ausklappen der Hornhaut als gleich groß angenommen werden und sich somit gegenseitig aufheben. Folglich resultiert ein intraokularer Druck aus einem Mittelwert der zum Einklappen und Ausklappen der Hornhaut aufgewendeten Kraft, aufgebracht durch den Luftstoß.

Alternativ ist es bekannt, eine Hysterese zwischen dem erstem und dem zweitem Applanationspunkt zu bestimmen und auf Basis der Hysteresemessung den intraokularen Druck abzuleiten bzw. zu korrigieren. Bei der Hysteresemessung wird der erste und zweite Applanationspunkt optisch detektiert und zum Zeitverlauf einer Druckkurve einer Pumpe in Relation gesetzt, das heißt für jeden Applanationspunkt wird ein zugehöriger Zeitwert sowie ein Druckwert ermittelt. Da ein Einklappen der Hornhaut bzw. ein Erreichen des ersten Applanationspunktes bei einem höheren Druckwert erfolgt, als ein Ausklappen bzw. ein Erreichen des zweiten Applanationspunktes, kann dieser Druckunterschied zur Bestimmung der Hysterese als eine Materialeigenschaft der Hornhaut herangezogen werden.

Nachteilig bei diesen Messverfahren ist, dass eine durch einen Luftstoß bewirkte Bewegung der Hornhaut dynamischen Effekten unterliegt, welche derartige Zeit-/Druckmessungen verfälschen können, insbesondere da die dynamischen Effekte bei den beschriebenen Non-Kontakt-Tonometermessungen nicht berücksichtigt werden können. Um derartige, unerwünschte Schwingungen der Hornhaut zu vermeiden, wird eine Geschwindigkeit des Luftstoßes soweit als Möglich minimiert, um ein Messergebnis nicht durch eine unerwünschte Hornhautbewegung zu verfälschen. Weiter ist es notwendig eine Synchronität zwischen Beginn des Luftstoßes und der erforderlichen Zeitmessung herzustellen. Durch eine mechanische, zur Ausbildung eines Luftstoßes verwendeten Pumpe, wie beispielsweise eine Kolbenpumpe, kann diese Zeitsynchronität jedoch, beispielsweise infolge von Massenträgheit oder -reibung, nicht genau erreicht und somit ein Messergebnis verfälscht werden. Auch wird, wie zuvor bereits erwähnt, der Luftstoß drucküberwacht, das heißt nach Bedarf im Verlauf der Messung beeinflusst. So wird nach einem Überschreiten des ersten Applanationspunktes der Luftstoß vermindert bzw. abgeschaltet, um ein übermäßiges Eindrücken der Hornhaut zu verhindern. Dazu ist jedoch ebenfalls eine kontinuierliche Drucküberwachung eines Pumpendrucks sowie eine Überwachung eines zeitlichen Verlaufs desselben relativ zu den Zeitpunkten der ersten und zweiten Applanation notwendig, was eine Einbeziehung einer Reihe von möglichen, einer Messung verfälschenden Fehlerquellen, zur Folge hat. Insgesamt sind daher die aus dem Stand der Technik bekannten Analyseverfahren und -systeme mit voneinander abhängiger, paralleler Druck- und Zeitmessung bei gleichzeitiger Detektion der Applanationspunkte gegenüber einer Messung mit einem Kontakt-Tonometer noch vergleichsweise ungenau.

Aus der US 2005/0030473 A1 ist ein Verfahren zur Messung eines intraokularen Drucks eines Auges mittels eines ophthalmologischen Analysegerätes bekannt. Bei der Berechnung eines intraokularen Drucks wird insbesondere ein Korrekturfaktor herangezogen, der einen Widerstand der Hornhaut gegen eine Auslenkung beschreiben soll und der in Abhängigkeit einer Dicke und von viskoelastischen Eigenschaften der Hornhaut variiert. Weiter kann eine diesbezügliche Steifigkeit der Hornhaut aus einer mit einem Non-Kontakt-Tonometer auf die Hornhaut aufgebrachten Kraft bzw. einem Luftstoß und einer dadurch erzeugten Applanationsfläche abgeleitet werden. Eine Verformung des Auges wird mit einer Hochgeschwindigkeitskamera aufgenommen, die seitlich neben dem Auge angeordnet ist. Da die Hornhaut transparent ist, können seitliche Schnittbilder der Hornhaut gewonnen werden.

Der Artikel "Understanding eye deformation in non-contact tonometry", veröffentlicht im Rahmen der "EMBS Annual International Conference, New York, 30.8. - 3.9.2006", beschreibt eine seitliche Videoaufnahme einer mit einem Non-Kontakt-Tonometer verformten Hornhaut eines Auges. Insbesondere wird ein Einfluss einer Steifigkeit der Hornhaut auf die Messergebnisse erwähnt. Weiter ist bekannt, dass eine Bewegung des gesamten Auges an einer Außengrenze der Hornhaut detektierbar ist.

Der vorliegenden Erfindung liegt daher die Aufgabe zugrunde, ein ophthalmologisches Analyseverfahren zur Messung eines intraokularen Drucks in einem Auge sowie ein derartiges Analysesystem vorzuschlagen, mit dem eine vergleichsweise verbesserte Messgenauigkeit erzielbar ist.

Diese Aufgabe wird durch ein ophthalmologisches Analyseverfahren mit den Merkmalen des Anspruchs 1 und ein Analysesystem mit den Merkmalen des Anspruchs 13 gelöst.

Bei dem erfindungsgemäßen, ophthalmologischen Analyseverfahren zur Messung eines intraokularen Drucks in einem Auge mit einem Analysesystem, umfasst das Analysensystem eine Betätigungseinrichtung, mit der eine Hornhaut des Auges berührungsfrei verformt wird, wobei mit der Betätigungseinrichtung ein Luftstoß zur Verformung der Hornhaut auf das Auge aufgebracht wird, ein Beobachtungssystem, mit dem die Verformung der Hornhaut beobachtet und aufgenommen wird, wobei mit dem Beobachtungssystem Schnittbilder der unverformten und verformten Hornhaut aufgenommen werden, und eine Analyseeinrichtung, mit der aus den Schnittbildern der Hornhaut der intraokulare Druck abgeleitet wird, wobei in der Analyseeinrichtung aus den Schnittbildern der Hornhaut eine Materialeigenschaft der Hornhaut abgeleitet wird, wobei eine maximale Verformung der Hornhaut aus den Schnittbildern der Hornhaut abgeleitet wird, wobei als eine Materialeigenschaft ein Durchmesser d₁ einer ersten Applanationsfläche der Hornhaut und ein Durchmesser d₂ einer von der ersten Applanationsfläche abweichenden Verformungsfläche der Hornhaut bei der maximalen Verformung der Hornhaut relativ zum Apex der Hornhaut abgeleitet wird, wobei die erste Applanationsfläche im Bereich einer Applanationsebene orthogonal zu einer Sehachse des Auges oder einer Geräteachse des Analysesystem liegt, wobei der Durchmesser d₂ einem Abstand von zwei gegenüberliegenden Punkten einer Längsschnittebene der Hornhaut in Richtung der Sehachse entspricht, wobei die Punkte jeweils die dem Analysesystem in der Längsschnittebene am nächsten zugewandten Punkte repräsentieren, wobei der intraokulare Druck unter Berücksichtigung der Durchmesser d₁ und d₂ der Hornhaut abgeleitet wird, wobei der intraokulare Druck durch Vergleich des ermittelten Durchmesser d₂ relativ zum Durchmesser d₁ mit in einer Datenbank gespeicherten Werten abgeleitet wird.

Bei der Verformung der Hornhaut mittels des Luftstoßes kommt es zu einer vollständigen Applanation der Hornhaut, wobei die erste Applanationsfläche mit dem Durchmesser d₁ ausgebildet wird. Die Applanationsfläche ist im Wesentlichen eben und liegt im Bereich einer Applanationsebene orthogonal zu einer Sehachse des Auges bzw. einer Geräteachse eines Analysesystems. Während der Verformung der Hornhaut wird in der Hornhaut eine konkave Vertiefung ausgebildet, die sich von der ersten Applanationsfläche wesentlich unterscheidet. Ein Vergleich der von der ersten Applanationsfläche abweichenden Verformungsfläche der Vertiefung mit der ersten Applanationsfläche ermöglicht eine Definition der Materialeigenschaft der Hornhaut, da die Verformungsfläche in Abhängigkeit der Materialeigenschaft ausgebildet wird. Die Materialeigenschaft betrifft somit keine allgemein bekannte Materialeigenschaft, sondern beruht alleine auf einer Definition zweier voneinander abweichender Geometrien der verformten Hornhaut. Ein Referenzmaßstab für die Abweichung ist dabei die erste Applanationsfläche bzw. der Durchmesser d₁ der ersten Applanationsfläche. Dadurch, dass der Vergleich mit dem Durchmesser dₙ der Verformungsfläche der Hornhaut erfolgt, kann der Vergleich besonders einfach ausgeführt werden. Der Durchmesser dₙ ist insbesondere bei einer Verformungsbewegung der Hornhaut nach Passieren der ersten Applanationsfläche bzw. eines ersten Applanationspunktes besonders einfach bestimmbar, da dann die Verformungsfläche eine konkave Gestalt annimmt. Weiter ist vorgesehen, dass die Verformungsfläche bzw. der Durchmesser dₙ in einem bestimmten Zeitabschnitt der Verformung relativ zur ersten Applanationsfläche oder auch ein anderer, messbarer Punkt oder eine Position der Hornhaut während der Verformung zur Definition der abweichenden Verformungsfläche der Hornhaut herangezogen wird. Weiter werden die ermittelte Abweichung und die Relativwerte der betreffenden Durchmesser in einer Datenbank gespeichert und verglichen. So ist für die in der Datenbank gespeicherten Werte ein objektiver Augeninnendruck oder auch ein entsprechender Korrekturwert bekannt, so dass der objektive intraokulare Druck des gemessenen Auges unter Berücksichtigung der geometrisch definierten Materialeigenschaft der Hornhaut abgeleitet wird.

Weiter ist zu beachten, dass bei dem Verfahren eine Druckmessung eines Pumpendrucks nicht notwendig sein muss. So kann jede beliebige Messung eines intraokularen Drucks immer mit dem gleichen, konstanten Pumpendruck durchgeführt werden. Da hier keine Variation einer Höhe des Pumpendrucks bzw. eine Zeitsynchronisation des Pumpendrucks erfolgen muss, können eine Reihe von möglichen Fehlerquellen ausgeschlossen und eine besonders genaue Messung durchgeführt werden. Im Übrigen kann die so bestimmte Materialeigenschaft der Hornhaut auch im Rahmen der refraktiven Augenchirurgie genutzt werden, um beispielsweise bei einen LASIK-Eingriff auf die jeweilige Hornhauteigenschaften abzustimmen.

Weiter ist es vorteilhaft, wenn ein Pumpendruck zur Erzeugung des Luftstoßes relativ zu einer Zeitdauer desselben in Form einer Glockenkurve verläuft. So kann der Pumpendruck für jede individuelle Messung identisch und vollkommen unbeeinflusst in Form des Luftstoßes auf die Hornhaut einwirken. Die Glockenkurve kann dabei unter anderem eine symmetrische Gestalt aufweisen.

Auch kann ein maximaler Pumpendruck zur Erzeugung des Luftstoßes bei vorangehenden und nachfolgenden Messungen gleich sein. So kann eine besonders gute Vergleichbarkeit von verschiedenen Messungen ermöglicht werden. Der maximale Pumpendruck kann beispielsweise 70 mm Hg betragen.

Um einen Pumpendruck dennoch gegebenenfalls korrigieren zu können bzw. um einen gewünschten Druckverlauf zu überprüfen, kann ein Pumpendruck zur Erzeugung des Luftstoßes bei Erreichen eines Applanationspunktes der Hornhaut gemessen werden. Beispielsweise kann eine Pumpe über einen Drucksensor verfügen, der die Überwachung des Pumpendrucks während der gesamten Messung ermöglicht. Dadurch können mögliche Fehler hinsichtlich des Pumpendrucks bei der Messung ausgeschlossen und eine Kontinuität aufeinanderfolgender Messungen sichergestellt werden.

Zur Ableitung der Materialeigenschaft kann auch ein Zeitabschnitt zwischen Beginn und Ende der Verformung der Hornhaut gemessen werden. So können insbesondere alle aufgenommenen Schnittbilder jeweils einem bestimmten Zeitpunkt der Messung zugeordnet werden, wodurch ein zeitlicher Ablauf der Verformung nachvollziehbar wird. Insbesondere kann ein Zeitpunkt der ersten und der zweiten Applanation der Hornhaut und somit ein zeitlicher Abstand genau bestimmt werden. So kann auch die Ermittlung dieses Zeitabschnittes zur Bestimmung der betreffenden Materialeigenschaft ausreichend sein. Weiter kann zur Ableitung der Materialeigenschaft ein Zeitabschnitt der gesamten Verformung der Hornhaut herangezogen werden.

Zur Ableitung der Materialeigenschaft kann weiter eine Geschwindigkeit der bewegten Hornhaut gemessen werden. Wenn insbesondere der Zeitverlauf einer Verformung der Hornhaut bekannt ist, kann so auch eine Dynamik der Verformung untersucht werden, um besondere dynamische Effekte bei der Verformung in Hinblick auf die betreffende Materialeigenschaft zu bewerten. Beispielsweise kann sich ein Nachschwingen der Hornhaut bei einem Luftstoß somit nicht mehr verfälschend auf ein Messergebnis auswirken, wenn das Nachschwingen bei der Messung berücksichtigt wird. Auch wird so eine Geschwindigkeit eines Luftstoßes in Bezug auf sonst unerwünschte dynamische Effekte für eine Messung beliebig wählbar. Auch ist es möglich von der gemessenen Geschwindigkeit auf eine Eindrücktiefe bzw. maximale Amplitude zu schließen, da zwischen diesen Größen ein funktionaler Zusammenhang besteht.

Um die Materialeigenschaft noch genauer zu bestimmen, wird erfindungsgemäß zur Ableitung der Materialeigenschaft eine maximale Verformung der Hornhaut aus den Schnittbildern der Hornhaut abgeleitet. Folglich kann eine maximale Eindrücktiefe der Hornhaut aus den Schnittbildern der Hornhaut ermittelt werden, wobei ergänzend auch ein Zeitpunkt der maximalen Verformung der Hornhaut zumindest relativ zu einem der Applanationspunkte festgestellt werden kann.

Zur Ableitung der Materialeigenschaft wird erfindungsgemäß ein Durchmesser d₂ einer Verformungsfläche der Hornhaut bei einer maximalen Verformung der Hornhaut in Richtung einer Sehachse oder Geräteachse bestimmt. Die maximale Verformung der Hornhaut kann aus einer Serie von Schnittbildern der verformten Hornhaut bestimmt werden. So wird es möglich für jede Messung einen Zeitpunkt der Verformung bzw. eine Geometrie der Hornhaut zu definieren, der bzw. die als ein Vergleichsmaßstab zur ersten Applanationsfläche der Hornhaut dienen kann. Auch kann der Durchmesser d₂ dann einfach dadurch bestimmt werden, dass dieser als ein Abstand von zwei gegenüberliegenden Punkten in einer Längsschnittebene der Hornhaut im Zustand der maximalen Verformung definiert wird, wobei die Punkte jeweils die dem Analysesystem am nähesten zugewandten Punkte repräsentieren. Diese Punkte können einem Schnittbild entnommen werden und repräsentieren folglich den Durchmesser d₂ der maximalen Verformung bzw. Deformation der Hornhaut.

Optional kann auch zur Ableitung der Materialeigenschaft bei Erreichen eines Applanationspunktes der Hornhaut eine Größe einer ebenen Applanationsfläche gemessen werden. Beispielsweise kann eine Größe der Applanationsfläche bzw. deren Durchmesser und/oder deren Form als ein Indikator für eine Steifigkeit der Hornhaut berücksichtigt werden. Auch können die an die jeweilige Applanationsfläche anschließenden Radien der Hornhaut als ein weiterer Indikator verwendet werden.

Zur Ableitung der Materialeigenschaft kann ein Verhältnis zwischen dem Durchmesser d₁ der ersten Applanationsfläche der Hornhaut und einem Durchmesser d₃ einer zweiten Applanationsfläche der Hornhaut bestimmt werden. Während einer Verformung der Hornhaut mittels des Luftstoßes erfolgt ein Einklappen der Hornhaut unter Ausbildung der ersten Applanationsfläche bis hin zu einer maximalen Verformung der Hornhaut mit einer konkaven Vertiefung sowie nachfolgend ein Ausklappen der Hornhaut unter Ausbildung der zweiten, weitestgehend ebenen Applanationsfläche bis zu einem Erreichen der ursprünglichen Gestalt der Hornhaut. Die zweite Applanationsfläche stellt damit einen in den Schnittbildern gut erkennbaren, geometrischen Referenzpunkt dar, der zur Definition der Materialeigenschaft durch einen Vergleich mit der ersten Applanationsfläche herangezogen werden kann. Insbesondere durch eine mögliche Abweichung der Durchmesser der Applanationsflächen kann eine Materialeigenschaft der Hornhaut definiert bzw. bestimmt werden.

Die Materialeigenschaft der Hornhaut kann noch genauer bestimmt werden, wenn eine Amplitude der Verformung der Hornhaut aus den Schnittbildern der Hornhaut abgeleitet wird. So kann der genaue geometrische Verlauf der Verformung leicht nachvollzogen werden. Das heißt, es kann zu jedem Zeitpunkt der Verformung die zu diesem Zeitpunkt existente, geometrische Gestalt der Verformung aufgenommen werden, so dass der geometrische Verlauf der Verformung in Art eines Films der Verformung erfasst werden kann. Beispielsweise ist auch ein Nachschwingen der Hornhaut nach einem Ausklappen bzw. dem zweiten Applanationspunkt so gut erfassbar.

Zur noch genaueren Ableitung der Materialeigenschaft kann eine Krümmung der unverformten und/oder verformten Hornhaut aus den Schnittbildern der Hornhaut abgeleitet werden. Da die Schnittbilder der Hornhaut auch eine Geometrie derselben beschreiben, insbesondere vor Aufbringen des Luftstoßes, kann die Geometrie der Hornhaut in Verbindung mit der betreffenden Materialeigenschaft der Hornhaut in die Berechnung des objektiven, intraokularen Drucks mit einbezogen werden. Das heißt die Krümmungsradien bzw. eine Kurvatur der Hornhaut kann auf einer äußeren und/oder inneren Hornhautoberfläche aus den Schnittbildern mittels Bildverarbeitung abgeleitet werden. Dabei können die Krümmungsradien bei der unverformten Hornhaut als ein Korrekturfaktor und beispielsweise die Dicke der Hornhaut bei der verformten Hornhaut in die Messung einfließen und als ein Indikator für die Materialeigenschaft herangezogen werden.

Die Materialeigenschaft der Hornhaut kann noch weiter differenziert werden, wenn die Verformung der Hornhaut durch eine freie Schwingung der Hornhaut fortgesetzt wird, und wenn als eine weitere Materialeigenschaft dann die freie Schwingung der Hornhaut bestimmt wird. Ein Ausschwingen der Hornhaut nach Aufbringen des Luftstoßes und Erreichen der ursprünglichen Gestalt der Hornhaut unterscheidet sich regelmäßig bei verschiedenen Augen. Somit kann das Ausschwingen der Hornhaut als eine weitere Materialeigenschaft der Hornhaut definiert werden, anhand der ein intraokularer Druck korrigiert werden kann. Folglich kann vorgesehen sein, mittels des Beobachtungssystems Schnittbilder der Hornhaut über die eigentliche Verformung der Hornhaut hinaus aufzunehmen, um das Ausschwingen bzw. die freie Schwingung der Hornhaut zu ermitteln.

Eine freie Schwingung der Hornhaut kann leicht dadurch bestimmt werden, dass eine Frequenz und/oder Amplitude der freien Schwingung gemessen wird. So wird es möglich die Frequenz und/oder eine Amplitudengröße und Abnahme während eines Ausschwingens zur Definition der weiteren Materialeigenschaft heranzuziehen.

Weiter kann als eine weitere Materialeigenschaft eine Steifigkeit der Hornhaut abgeleitet werden. Der Begriff der Steifigkeit ist dann explizit nicht als ein Elastizitätsmodul bzw. Youngscher-Modul zu verstehen, sondern als eine Materialeigenschaft, die durch eine auf das Auge wirkenden Druckbelastung charakterisiert ist bzw. dieser entgegensteht, also den bei einer Tonometermessung tatsächlich auftretenden Lastfall betrifft. Die Steifigkeit ist somit eine richtungsabhängige Kenngröße des Materials der Hornhaut. Weiter ist die Steifigkeit durch das Material der Hornhaut selbst und nicht durch sonstige, äußere Einflüsse bestimmt. Auch wirken im Material der Hornhaut intrinsische Spannungen die die Steifigkeit der Hornhaut beeinflussen.

So kann während einer einzigen Messung durch Aufbringen eines Luftstoßes nach einem herkömmlichen, tonometrischen Verfahren ein erster intraokularer Druck bestimmt werden. Parallel dazu kann aus der Verformung der Hornhaut, die während der Verformung durch das Beobachtungssystem aufgenommen wird, die Steifigkeit der Hornhaut abgeleitet werden. Da die Steifigkeit der Hornhaut einen wesentlichen Einfluss auf ein Verformungsverhalten der Hornhaut bzw. die Messung des ersten intraokularen Drucks des Auges hat, kann der Einfluss der Hornhaut auf die Messung des ersten intraokularen Drucks berücksichtigt werden. So kann der zuvor gemessene, erste intraokulare Druck um den Einfluss der Hornhaut auf die Messung korrigiert werden, so dass ein objektiver intraokularer Druck als ein Ergebnis der Messung abgeleitet wird. Die Steifigkeit der Hornhaut ist dabei im Wesentlichen näherungsweise eine lineare Funktion von dem ersten, gemessenen subjektiven Intraokularen Druck des Auges sowie einer gemessenen maximalen Amplitude der Verformung der Hornhaut. Bei einem Graph der Funktion der Steifigkeit kann der subjektive intraokulare Druck beispielsweise auf einer Ordinatenachse und die maximale Amplitude der Verformung auf einer Abzissenachse abgebildet sein, wobei die Steifigkeit dann im Wesentlichen eine Form einer Geraden mit negativer Steigung aufweist. Je nach Messwert für die Abzissenachse und Ordinatenachse ergibt sich bei sich verändernden Messwerten im Wesentlichen eine Parallelverschiebung der Geraden, woraus jeweils unterschiedliche Steifigkeiten resultieren. Der objektive intraokulare Druck wird von der ermittelten Steifigkeit abgeleitet bzw. kann von der Geraden der Steifigkeit aus einem Schnittpunkt des Wertes für den subjektiven intraokularen Drucks bzw. des Wertes für die maximale Amplitude mit der Geraden für die Steifigkeit abgetragen werden. Bei der Messung kann immer die Steifigkeit der Hornhaut als eine Materialeigenschaft für jede Messung neu ermittelt werden, dass heißt, es wird nicht, wie aus dem Stand der Technik bekannt, von einer für jedes beliebige Auge konstanten Materialeigenschaft ausgegangen. Alternativ können auch anstelle der Amplituden die Durchmesser der Applanationsfläche und der Verformungsfläche in dem Graph eingesetzt und analog verwendet werden.

Weiter kann es besonders vorteilhaft sein, wenn während der Messung bzw. des Verformungsvorgangs der Hornhaut eine Serie bzw. Vielzahl von Schnittbildern der Hornhaut aufgenommen wird. So wird es möglich, eine Verformung der Hornhaut im Detail zu verfolgen und mittels einer Bildverarbeitung der Schnittbilder aus dem Verformungsablauf die entsprechende Materialeigenschaft bzw. einen objektiven intraokularen Druck abzuleiten.

Weiter kann als eine weitere, vom intraokularen Druck unabhängige Materialeigenschaft eine Spannung der Hornhaut abgeleitet werden, wobei Spannungen im Material der Hornhaut visualisiert werden können. Die weitere Materialeigenschaft kann im vorliegenden Zusammenhang als eine Eigenschaft definiert sein, die dem Material unabhängig von äußeren Einflüssen innewohnt. Eine Struktureigenschaft ist eine Eigenschaft, die durch äußere Einflüsse im Material oder auch durch eine Gestalt des Materials beeinflusst ist. So kann es vorgesehen sein, Spannungen der Hornhaut durch die Aufnahme der Schnittbilder zu visualisieren. Dabei kann zwischen Spannungen unterschieden werden, die unabhängig von einem intraokularen Druck, abhängig von einem intraokularen Druck und bedingt durch die Verformung der Hornhaut im Material der Hornhaut vorliegen. Diese Unterscheidung wird dadurch möglich, dass durch die Aufnahme der Schnittbilder Spannungen der unverformten Hornhaut und nachfolgend Spannungen der verformten Hornhaut aufgenommen und visualisiert werden können. Je nach Art, Stärke, Richtung oder Verteilung der Spannungen in den Schnittbildern der Hornhaut, kann der intraokularen Druck unter Berücksichtigung der Spannung zu korrigiert und abgeleitet werden. Eine Korrektur des intraokularen Drucks ist insbesondere auch durch einen Vergleich eines Verhältnisses der Spannungen der unverformten und der verformten Hornhaut an einem definierten Punkt bzw. einer Position der verformten Hornhaut möglich. In einem weiteren Schritt des Verfahrens kann vorgesehen sein, die visualisierten Spannungen mit in einer Datenbank, gespeicherten, visualisierten Spannungen zu vergleichen, um den intraokularen Druck zu korrigieren. So kann für die in der Datenbank gespeicherten Werte ein objektiver Augeninnendruck oder auch ein entsprechender Korrekturwert bekannt sein, so dass der objektive intraokulare Druck des gemessenen Auges unter Berücksichtigung der Spannungen der Hornhaut abgeleitet werden kann.

Als ein Schnittbild kann jeweils eine spannungsoptische Darstellung der Hornhaut verwendet werden. Eine spannungsoptische Darstellung ermöglicht eine einfache Visualisierung einer Spannungsverteilung in lichtdurchlässigen Körpern, wobei eine Verteilung und Größe der mechanischen Spannung an allen Stellen der Hornhaut, oder auch in anderen lichtdurchlässigen Bereichen des Auges, einfach dargestellt und mittels Bildverarbeitung ausgewertet werden kann. Insbesondere können dabei in der Ebene des Schnittbildes auftretende Spannungen visualisiert werden. Quer zur Ebene des Schnittbildes verlaufende Spannungen werden dann nicht berücksichtigt, wobei dies zur Korrektur des intraokularen Drucks auch nicht zwingend notwendig ist.

Besonders einfach kann aus Spannungslinien der spannungsoptischen Darstellung die weitere Materialeigenschaft der Hornhaut abgeleitet werden. Die Spannungslinien sind besonders gut visuell erkennbar und auch wird es dann möglich zwischen der Materialeigenschaft der Hornhaut zu unterscheiden. Bei den Spannungslinien kann zwischen Isochromaten und Isoklinen unterschieden werden, wobei die Isochromaten Spannungslinien mit konstanter Hauptspannungsdifferenz sind und die Isoklinen Spannungstrajektorien der Hornhaut unter einer gegebenen Belastung repräsentieren. So können aus einer Vielzahl von während einer Verformung der Hornhaut gewonnen Schnittbildern, sich aufgrund der durch den Luftstoß bedingten Belastung der Hornhaut verändernde Spannungslinien und aufgrund einer Gestalt der Hornhaut selbst in dieser vorliegende Spannungslinien, welche sich relativ zur Hornhaut nicht wesentlich verändern, unterschieden werden.

Das Analysesystem kann dann in Art eines Polariskops ausgebildet sein, wobei das Beobachtungssystem dann eine Beleuchtungseinrichtung und eine Kameraeinrichtung umfassen kann, die jeweils einen Polarisator aufweisen. Beispielsweise kann es dann ausreichend sein an der Beleuchtungseinrichtung einen entsprechenden Polarisiationsfilter und an der Kameraeinrichtung einen Polarisationsfilter vorzusehen, um Spannungen im Material der Hornhaut visualisieren zu können.

Die Messung kann noch weiter dadurch verbessert werden, dass unterschiedlichen Bereichen der Hornhaut jeweils voneinander abweichende Materialeigenschaften zugeordnet werden. So können, bei angenommener, gleicher Dicke der Hornhaut die Materialeigenschaften in unterschiedlichen Bereichen eines Querschnitts der Hornhaut oder in Bezug auf einen Oberflächenbereich der Hornhaut variieren bzw. voneinander verschieden sein.

In einer vorteilhaften Ausführungsform des Analyseverfahrens kann das Beobachtungssystem eine Kamera und eine Beleuchtungseinrichtung in einer Scheimpfluganordnung umfassen, wobei dann mittels der Kamera die Schnittbilder aufgenommen werden können. Das heißt die Kamera kann relativ zu einer optischen Achse einer Spaltbeleuchtungseinrichtung zur Beleuchtung des Auges in einer Scheimpfluganordnung angeordnet sein, so dass ein beleuchtetes Querschnittsbild des Auges mit der Kamera aufgenommen werden kann. Eine Kamera kann beispielsweise auch als eine Hochgeschwindigkeitskamera verwendet werden, die mindestens 4000 Bilder pro Sekunde aufnehmen kann. Auch kann die optische Achse der Spaltbeleuchtungseinrichtung in eine Sehachse des Auges fallen bzw. mit dieser übereinstimmen. Vorzugsweise kann dann auch eine Wirkrichtung des Luftstoßes koaxial zur optischen Achse der Spaltbeleuchtungseinrichtung verlaufen.

Das erfindungsgemäße ophthalmologische Analysesystem zur Messung eines intraokularen Drucks in einem Auge umfasst eine Betätigungseinrichtung, mit der eine Hornhaut des Auges berührungsfrei verformt werden kann, wobei mit der Betätigungseinrichtung ein Luftstoß zur Verformung der Hornhaut auf das Auge aufgebracht werden kann, ein Beobachtungssystem, mit dem die Verformung der Hornhaut beobachtet und aufgenommen werden kann, wobei mit dem Beobachtungssystem Schnittbilder der unverformten und/oder verformten Hornhaut aufgenommen werden können, und eine Analyseeinrichtung, mit der aus den Schnittbildern der Hornhaut der intraokulare Druck abgeleitet werden kann, wobei in der Analyseeinrichtung aus den Schnittbildern der Hornhaut eine Materialeigenschaft der Hornhaut abgeleitet wird, wobei eine maximale Verformung der Hornhaut aus den Schnittbildern der Hornhaut abgeleitet wird, wobei als eine Materialeigenschaft ein Durchmesser d₁ einer ersten Applanationsfläche der Hornhaut und ein Durchmesser d₂ einer von der ersten Applanationsfläche abweichenden Verformungsfläche der Hornhaut bei der maximalen Verformung der Hornhaut relativ zum Apex der Hornhaut abgeleitet wird, wobei die erste Applanationsfläche im Bereich einer Applanationsebene orthogonal zu einer Sehachse des Auges oder einer Geräteachse des Analysesystem liegt, wobei der Durchmesser d₂ einem Abstand von zwei gegenüberliegenden Punkten einer Längsschnittebene der Hornhaut in Richtung der Sehachse entspricht, wobei die Punkte jeweils die dem Analysesystem in der Längsschnittebene am nächsten zugewandten Punkte repräsentieren, wobei der intraokulare Druck unter Berücksichtigung der Durchmesser d₁ und d₂ der Hornhaut abgeleitet wird, wobei der intraokulare Druck durch Vergleich des ermittelten Durchmesser d₂ relativ zum Durchmesser d₁ mit in einer Datenbank gespeicherten Werten ableitbar ist. Hinsichtlich der sich aus dem erfindungsgemäßen Analysesystem ergebenden vorteilhaften Wirkungen wird auf die Beschreibung des erfindungsgemäßen ophthalmologischen Analyseverfahrens verwiesen.

Nachfolgend wird eine bevorzugte Ausführungsform der Erfindung unter Bezugnahme auf die beigefügten Zeichnungen näher erläutert.

Es zeigen:
- **Fig. 1a** bis **1e:**: eine Verformung einer Hornhaut eines Auges während einer Messung in einer Längsschnittansicht;
- **Fig. 2:**: eine Diagrammdarstellung von Pumpendruck und -zeit während einer Messung;
- **Fig. 3:**: eine Diagrammdarstellung von gemessenem intraokularen Druck und Verformung der Hornhaut;
- **Fig. 4a** bis **4b:**: eine Visualisierung von Spannungen im Material der Hornhaut des Auges.

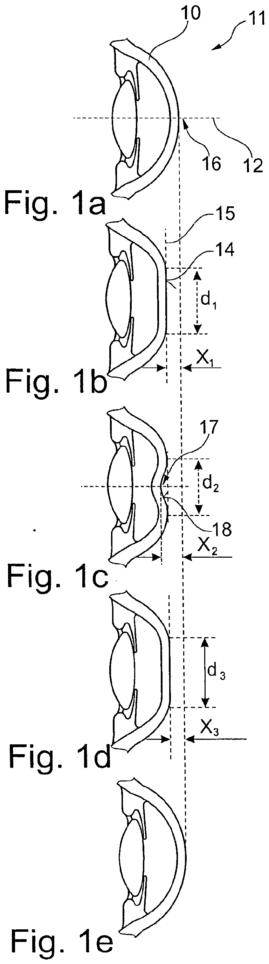

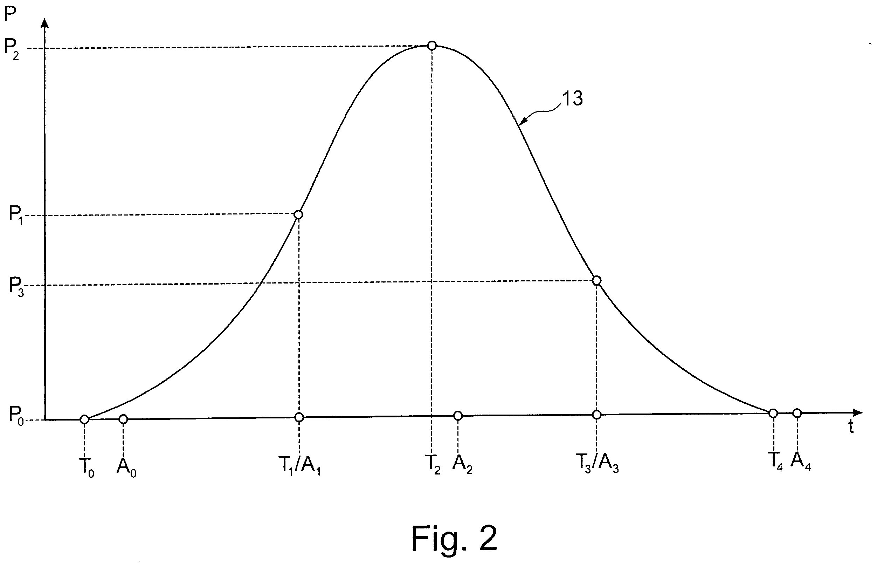

Die **Fig. 1a** bis **1e** zeigen ausgewählte Verformungszustände einer Hornhaut 10 eines Auges 11 während einer einzelnen Messung eines Augeninnendrucks durch ein hier nicht dargestelltes Analysesystem. Die Darstellungen sind jeweils Längsschnittdarstellungen entlang einer Sehachse 12 des Auges 11. **Fig. 2** ist eine Diagrammdarstellung mit einer Zeit t auf der Abzissenachse und einem Pumpendruck p auf der Ordinatenachse. Der Pumpendruck verläuft unabhängig vom Einsatz eines hier nicht dargestellten Beobachtungssystems bzw. einer Scheimpflugkamera mit einer Spaltbeleuchtungseinrichtung in Art einer symmetrischen Glockenkurve 13, beginnend mit einem Druck P₀ bei einem Startpunkt T₀ der Pumpe bis hin zu einem maximalen Pumpendruck P₂ zum Zeitpunkt T₂ und wieder abfallend bis hin zu dem Pumpendruck P₀ in einem Endpunkt T₄. Der durch den Start der Pumpe bei T₀ abgegebene Luftstoß auf die Hornhaut 10 führt zu einer ersten, mittels des Beobachtungssystems aufnehmbaren Verformung der Hornhaut 10 unmittelbar nach dem Zeitpunkt A₀. **Fig. la** repräsentiert die Gestalt der noch nicht verformten Hornhaut 10 zum Zeitpunkt A₀. Mit steigendem Pumpendruck kommt es zum Zeitpunkt A₁ zu einer vollständigen Applanation der Hornhaut 10 gemäß **Fig. 1b****,** wobei wie hier dargestellt, eine Applanationsfläche 14 mit einem Durchmesser d₁ ausgebildet wird, die im Wesentlichen eben ist und in einer Applanationsebene 15 liegt. Die Hornhaut ist dann um ein Maß X₁ vom Apex 16 der Hornhaut 10 entfernt bzw. eingedrückt. Optional und nicht notwendigerweise kann bei diesem sogenannten ersten Applanationspunkt zum Zeitpunkt A₁ ein Pumpendruck P₁ zum übereinstimmenden Zeitpunkt T₁ ermittelt werden. Nach Erreichen des Pumpendrucks P₂ kommt es zu einer maximalen Verformung der Hornhaut 10 zum Zeitpunkt A₂ entsprechend der Darstellung in **Fig. 1c****.** Ein eine maximale Verformung bestimmender Punkt 17 ist dabei um ein Maß X₂ vom Apex 16 der Hornhaut 10 entfernt. Es handelt sich in diesem Fall also um eine maximale Auslenkung einer Amplitude der Verformung. Bei dieser maximalen Amplitude der Verformung wird ein Durchmesser d₂ einer konkaven Verformungsfläche 18 ausgebildet und erfasst. Der Durchmesser d₂ ist durch einen Abstand von zwei gegenüberliegenden Punkten einer Längsschnittebene der Hornhaut 10 definiert, wobei die Punkte jeweils die dem Analysesystem am nächsten zugewandten Punkte der Hornhaut 10 repräsentieren. Nachfolgend kommt es zu einer Rückbewegung bzw. einem Ausschwingen der Hornhaut 10, wobei zum Zeitpunkt A₃ der sogenannte zweite Applanationspunkt gemäß Darstellung in **Fig. 1d** erreicht wird. Hier wird ebenfalls ein Durchmesser d₃ sowie ein Abstand X₃ erfasst. Auch ist es optional möglich einen Pumpendruck P₃ zum übereinstimmenden Zeitpunkt T₃ zu bestimmen. Nach dem Rückgang des Pumpendrucks auf den Ursprungswert P₀ zum Zeitpunkt T₄ gelangt die Hornhaut 10 zum Zeitpunkt A₄ ebenfalls wieder in ihre in **Fig. le** dargestellte Ausgangslage. Entsprechend der vorangehenden Beschreibung einer einzelnen Messung eines intraokularen Drucks eines Auges werden die in den **Fig. 1a** bis **1e** dargestellten Verformungszustände der Hornhaut 10, welche durch die jeweiligen mit A₀ bis A₄ gekennzeichneten Zeitpunkte charakterisiert sind, ermittelt. Dabei werden insbesondere unabhängig von einem Pumpendruck p Zeitabstände der betreffenden Zeitpunkte A₀ bis A₄ sowie die Maße bzw. Eindrücktiefen X₁, X₂ und X₃ erfasst, wobei aus diesen Kenngrößen eine Steifigkeit der Hornhaut 10 abgeleitet wird. Ein gemessener intraokularer Druck wird dann durch einen durch die Steifigkeit der Hornhaut bestimmten Wert korrigiert, sodass ein objektiver intraokularer Druck als ein Ergebnis der Messung ausgegeben wird.

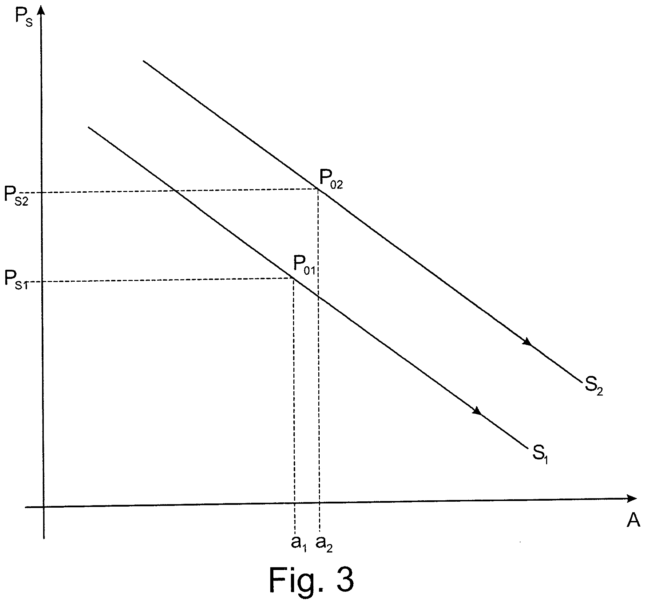

**Fig. 3** zeigt eine Diagrammdarstellung mit einem subjektiven, gemessenen intraokularen Druck auf der Ordinatenachse und einer Auslenkung einer Amplitude einer maximalen Verformung der Hornhaut 10 auf der Abzissenachse. Bei beispielsweise einem subjektiven intraokularen Druck auf Pₛ₁ und einer Amplitude a₁, welche dem Abstand X₂ entspricht, ergibt sich eine Steifigkeit S₁ als eine im Wesentlichen lineare Funktion mit negativer Steigung. Die Steifigkeit S₁ kann jedoch auch von einer linearen Funktion abweichen und als eine Linie mit einem vergleichsweise großen Krümmungsradius ausgebildet sein. Ein objektiver intraokularer Druck Pₒ₁ kann von der durch die Steifigkeit S₁ definierten Geraden als eine Variable entnommen werden. Analog dazu ergibt sich bei einem Druck Pₛ₂ und einer Auslenkung a₂ eine Parallelverschiebung der Geraden mit einer Steifigkeit S₂, woraus wieder ein objektiver intraokularer Druck Pₒ₂ abgeleitet werden kann. Alternativ können auch anstelle der Amplituden a₁ und a₂ die Durchmesser d₁ und d₂ in dem Diagramm eingesetzt und analog verwendet werden.

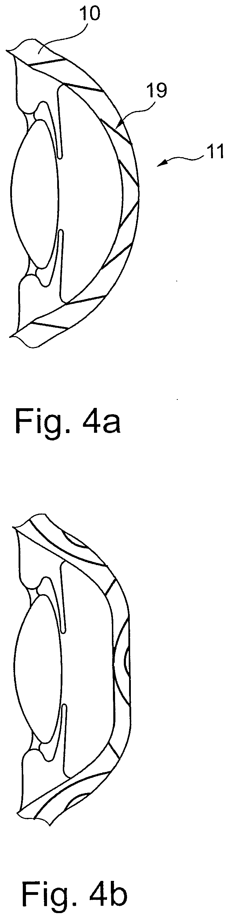

Die **Fig. 4a** bis **4b** zeigen die Verformungszustände der Hornhaut 10 des Auges 11 analog den **Fig. 1a** und **1b****.** Im Unterschied dazu sind bei den **Fig. 4a** und **4b** Spannungen im Material der Hornhaut visualisiert. So sind insbesondere Spannungslinien 19 im Material der Hornhaut 10 sichtbar, die Hauptspannungen längs und quer der Sehachse 12 repräsentieren. Die **Fig. 4a** zeigt somit Spannungen im Auge 11 in einem Ruhezustand der Hornhaut 10 und die **Fig. 4b** Spannungen im Auge 11 der verformten Hornhaut 10, die sich von den Spannungen im Ruhezustand wesentlich unterscheiden. Ein Vergleich der Spannung auf Basis der Spannungslinien 19 ermöglicht somit eine Definition einer Struktur- und/oder Materialeigenschaft der Hornhaut, die zur Korrektur eines gemessenen intraokularen Drucks und damit zur Ableitung eines objektiven intraokularen Drucks herangezogen werden kann.

## Patentansprüche

1. Ophthalmologisches Analyseverfahren zur Messung eines intraokularen Drucks in einem Auge (11) mit einem Analysesystem, gebildet aus einer Betätigungseinrichtung, mit der eine Hornhaut (10) des Auges berührungsfrei verformt wird, wobei mit der Betätigungseinrichtung ein Luftstoß zur Verformung der Hornhaut auf das Auge aufgebracht wird, einem Beobachtungssystem, mit dem die Verformung der Hornhaut beobachtet und aufgenommen wird, wobei mit dem Beobachtungssystem Schnittbilder der unverformten und verformten Hornhaut aufgenommen werden, und einer Analyseeinrichtung, mit der aus den Schnittbildern der Hornhaut der intraokulare Druck abgeleitet wird, wobei in der Analyseeinrichtung aus den Schnittbildern der Hornhaut eine Materialeigenschaft der Hornhaut abgeleitet wird,
**dadurch gekennzeichnet,**
**dass** eine maximale Verformung der Hornhaut aus den Schnittbildern der Hornhaut abgeleitet wird, wobei als eine Materialeigenschaft ein Durchmesser d₁ einer ersten Applanationsfläche (14) der Hornhaut und ein Durchmesser d₂ einer von der ersten Applanationsfläche abweichenden Verformungsfläche (18) der Hornhaut bei der maximalen Verformung der Hornhaut relativ zum Apex (16) der Hornhaut abgeleitet wird, wobei die erste Applanationsfläche im Bereich einer Applanationsebene (15) orthogonal zu einer Sehachse (12) des Auges oder einer Geräteachse des Analysesystem liegt, wobei der Durchmesser d₂ einem Abstand von zwei gegenüberliegenden Punkten einer Längsschnittebene der Hornhaut in Richtung der Sehachse entspricht, wobei die Punkte jeweils die dem Analysesystem in der Längsschnittebene am nächsten zugewandten Punkte der Hornhaut repräsentieren, wobei der intraokulare Druck unter Berücksichtigung der Durchmesser d₁ und d₂ der Hornhaut abgeleitet wird, wobei der intraokulare Druck durch Vergleich der Relativwerte der betreffenden Durchmesser mit in einer Datenbank gespeicherten Werten abgeleitet wird.

2. Analyseverfahren nach Anspruch 1,
**dadurch gekennzeichnet,**
**dass** ein Pumpendruck zur Erzeugung des Luftstoßes relativ zu einer Zeitdauer desselben in Form einer Glockenkurve (13) verläuft.

3. Analyseverfahren nach Anspruch 1 oder 2,
**dadurch gekennzeichnet,**
**dass** ein maximaler Pumpendruck zur Erzeugung des Luftstoßes bei vorangehenden und nachfolgenden Messungen gleich ist.

4. Analyseverfahren nach einem der vorangehenden Ansprüche,
**dadurch gekennzeichnet,**
**dass** ein Pumpendruck zur Erzeugung des Luftstoßes bei Erreichen eines Applanationspunktes der Hornhaut (10) gemessen wird.

5. Analyseverfahren nach einem der vorangehenden Ansprüche,
**dadurch gekennzeichnet,**
**dass** eine Geschwindigkeit der bewegten Hornhaut (10) gemessen wird, wobei die Geschwindigkeit zur Ableitung der Materialeigenschaft gemessen wird.

6. Analyseverfahren nach einem der vorangehenden Ansprüche,
**dadurch gekennzeichnet,**
**dass** bei Erreichen eines Applanationspunktes der Hornhaut (10) eine Größe einer ebenen Applanationsfläche (14, 18) gemessen wird, wobei die Größe zur Ableitung der Materialeigenschaft gemessen wird.

7. Analyseverfahren nach einem der vorangehenden Ansprüche,
**dadurch gekennzeichnet,**
**dass** ein Verhältnis zwischen dem Durchmesser d₁ der ersten Applanationsfläche (14) der Hornhaut (10) und einem Durchmesser d₃ einer zweiten Applanationsfläche der Hornhaut bestimmt wird, wobei das Verhältnis zur Ableitung der Materialeigenschaft bestimmt wird.

8. Analyseverfahren nach einem der vorangehenden Ansprüche,
**dadurch gekennzeichnet,**
**dass** eine Amplitude eines geometrischen Verformungsverlaufs der Hornhaut (10) aus den Schnittbildern der Hornhaut abgeleitet wird, wobei die Amplitude zur Ableitung der Materialeigenschaft abgeleitet wird.

9. Analyseverfahren nach einem der vorangehenden Ansprüche,
**dadurch gekennzeichnet,**
**dass** eine Krümmung der unverformten und/oder verformten Hornhaut (10) aus den Schnittbildern der Hornhaut abgeleitet wird, wobei die Krümmung zur Ableitung der Materialeigenschaft abgeleitet wird.

10. Analyseverfahren nach einem der vorangehenden Ansprüche,
**dadurch gekennzeichnet,**
**dass** die Verformung der Hornhaut (10) durch eine freie Schwingung der Hornhaut fortgesetzt wird, und dass als eine weitere Materialeigenschaft die freie Schwingung der Hornhaut bestimmt wird.

11. Analyseverfahren nach Anspruch 10,
**dadurch gekennzeichnet,**
**dass** eine Frequenz und/oder Amplituden der freien Schwingung gemessen werden.

12. Analyseverfahren nach einem der vorangehenden Ansprüche,
**dadurch gekennzeichnet,**
**dass** das Beobachtungssystem eine Kamera und eine Beleuchtungseinrichtung in einer Scheimpfluganordnung umfasst, wobei mittels der Kamera die Schnittbilder aufgenommen werden.

13. Ophthalmologisches Analysesystem zur Messung eines intraokularen Drucks in einem Auge (11), umfassend eine Betätigungseinrichtung, mit der eine Hornhaut (10) des Auges berührungsfrei verformt werden kann, wobei mit der Betätigungseinrichtung ein Luftstoß zur Verformung der Hornhaut auf das Auge aufgebracht werden kann, ein Beobachtungssystem, mit dem die Verformung der Hornhaut beobachtet und aufgenommen werden kann, wobei mit dem Beobachtungssystem Schnittbilder der unverformten und verformten Hornhaut aufgenommen werden können, und eine Analyseeinrichtung, mit der aus den Schnittbildern der Hornhaut der intraokulare Druck abgeleitet werden kann, wobei die Analyseeinrichtung dazu eingerichtet ist, aus den Schnittbildern der Hornhaut eine Materialeigenschaft der Hornhaut abzuleiten, **dadurch gekennzeichnet,**
**dass** eine maximale Verformung der Hornhaut aus den Schnittbildern der Hornhaut abgeleitet wird, wobei als eine Materialeigenschaft ein Durchmesser d₁ einer ersten Applanationsfläche (14) der Hornhaut und ein Durchmesser d₂ einer von der ersten Applanationsfläche abweichenden Verformungsfläche (18) der Hornhaut bei der maximalen Verformung der Hornhaut relativ zum Apex (16) der Hornhaut abgeleitet wird, wobei die erste Applanationsfläche im Bereich einer Applanationsebene (15) orthogonal zu einer Sehachse (12) des Auges oder einer Geräteachse des Analysesystem liegt, wobei der Durchmesser d₂ einem Abstand von zwei gegenüberliegenden Punkten einer Längsschnittebene der Hornhaut in Richtung der Sehachse entspricht, wobei die Punkte jeweils die dem Analysesystem in der Längsschnittebene am nächsten zugewandten Punkte der Hornhaut repräsentieren, wobei der intraokulare Druck unter Berücksichtigung der Durchmesser d₁ und d₂ der Hornhaut abgeleitet wird, wobei der intraokulare Druck durch Vergleich der Relativwerte der betreffenden Durchmesser mit in einer Datenbank gespeicherten Werten ableitbar ist.

## Claims

1. An ophthalmological analysis method for measuring an intraocular pressure in an eye (11) using an analysis system consisting of an actuating device with which a cornea (10) of the eye is deformed in a contactless manner, wherein the actuating device causes an air puff to be applied to the eye in such a manner that the cornea is deformed, an observation system with which the deformation of the cornea is observed and recorded, wherein sectional images of the cornea when it is not deformed or deformed are created with the observation system, and an analysis device with which the intraocular pressure is derived from the sectional images of the cornea, wherein a material characteristic of the cornea is derived in the analysis device from the sectional images of the cornea,
**characterised in that**
a maximum deformation of the cornea is derived from the sectional images of the cornea, wherein a diameter d₁ of a first applanation area (14) of the cornea and a diameter d₂ of a deformation area (18) of the cornea differing from the first applanation area are derived as a material characteristic for the maximum deformation of the cornea relative to the apex (16) of the cornea, wherein the first applanation area lies orthogonally to an optical axis (12) of the eye or to a device axis of the analysis system in the region of an applanation plane (15), wherein the diameter d₂ corresponds to a distance between two opposite points of a longitudinal sectional plane of the cornea in the direction of the optical axis, wherein each of the points represents the points of the cornea closest to the analysis system in the longitudinal sectional plane, wherein the intraocular pressure is derived taking the diameters d₁ and d₂ of the cornea into account, wherein the intraocular pressure is derived by comparing the relative values of the corresponding diameters with values stored in a database.

2. The analysis method as recited in claim 1,
**characterised in that**
a pump pressure for producing the air puff progresses in the form of a bell curve (13) relative to a duration of the air puff.

3. The analysis method as recited in claim 1 or 2,
**characterised in that**
a maximum pump pressure for producing the air puff is the same in preceding and following measurements.

4. The analysis method as recited in any of the preceding claims,
**characterised in that**
a pump pressure for producing the air puff is measured when an applanation point of the cornea (10) is reached.

5. The analysis method as recited in any of the preceding claims,
**characterised in that**
a speed of movement of the cornea (10) is measured, wherein the speed is measured for the purpose of deriving the material characteristic.

6. The analysis method as recited in any of the preceding claims,
**characterised in that**
a size of a flat applanation area (14, 18) is measured when an applanation point of the cornea (10) is reached, wherein the size is measured for the purpose of deriving the material characteristic.

7. The analysis method as recited in any of the preceding claims,
**characterised in that**
a ratio between the diameter d₁ of the first applanation area (14) of the cornea (10) and a diameter d₃ of a second applanation area of the cornea is determined, wherein the ratio is determined for the purpose of deriving the material characteristic.

8. The analysis method as recited in any of the preceding claims,
**characterised in that**
an amplitude of a geometrical progression of the deformation of the cornea (10) is derived from the sectional images of the cornea, wherein the amplitude is derived for the purpose of deriving the material characteristic.

9. The analysis method as recited in any of the preceding claims,
**characterised in that**
a curvature of the cornea (10) with and/or without deformation is derived from the sectional images of the cornea, wherein the curvature is derived for the purpose of deriving the material characteristic.

10. The analysis method as recited in any of the preceding claims,
**characterised in that**
the deformation of the cornea (10) is continued by a free oscillation of the cornea and that the free oscillation of the cornea is determined as a further material characteristic.

11. The analysis method as recited in claim 10,
**characterised in that**
a frequency and/or amplitude of the free oscillation is/are measured.

12. The analysis method as recited in any of the preceding claims,
**characterised in that**
the observation system comprises a camera and an illumination device in a Scheimpflug arrangement, wherein the sectional images are taken with the camera.

13. An ophthalmological analysis system for measuring an intraocular
pressure in an eye (11), comprising an actuating device with which a cornea (10) of the eye can be deformed in a contactless manner, wherein the actuating device causes an air puff to be applied to the eye in such a manner that the cornea is deformed, an observation system with which the deformation of the cornea can be observed and recorded, wherein sectional images of the cornea when it is not deformed or deformed can be created with the observation system, and an analysis device with which the intraocular pressure can be derived from the sectional images of the cornea, wherein the analysis device is designed for deriving a material characteristic of the cornea from the sectional images of the cornea,
**characterised in that**
a maximum deformation of the cornea is derived from the sectional images of the cornea, wherein a diameter d₁ of a first applanation area (14) of the cornea and a diameter d₂ of a deformation area (18) of the cornea differing from the first applanation area are derived as a material characteristic for the maximum deformation of the cornea relative to the apex (16) of the cornea, wherein the first applanation area lies orthogonally to an optical axis (12) of the eye or to a device axis of the analysis system in the region of an applanation plane (15), wherein the diameter d₂ corresponds to a distance between two opposite points of a longitudinal sectional plane of the cornea in the direction of the optical axis, wherein each of the points represents the points of the cornea closest to the analysis system in the longitudinal sectional plane, wherein the intraocular pressure is derived taking the diameters d₁ and d₂ of the cornea into account, wherein the intraocular pressure is derived by comparing the relative values of the corresponding diameters with values stored in a database.

## Revendications

1. Procédé d'analyse ophtalmologique destiné à mesurer une pression intraoculaire dans un œil (11) à l'aide d'un système d'analyse, consistant en une unité d'actionnement à l'aide de laquelle une cornée (10) de l'œil est déformée sans contact, ladite unité d'actionnement dirigeant un jet d'air sur l'œil pour déformer la cornée, en un système d'observation à l'aide duquel la déformation de la cornée est observée et acquise, ledit système d'observation acquérant des images en coupe de la cornée non déformée et déformée, et en une unité d'analyse à l'aide de laquelle la pression intraoculaire est dérivée des images en coupe de la cornée, une propriété du matériau de la cornée étant dérivée des images en coupe de la cornée dans l'unité d'analyse,
**caractérisé en ce**
**qu'**une déformation maximale de la cornée est dérivée des images en coupe de la cornée, un diamètre d₁ d'une première surface d'applanation (14) de la cornée et un diamètre d₂ d'une surface de déformation (18) de la cornée différant de la première surface d'applanation étant dérivés comme propriété de matériau pour la déformation maximale de la cornée par rapport à l'apex (16) de la cornée, ladite première surface d'applanation étant orthogonale par rapport à un axe visuel (12) de l'œil ou à un axe de dispositif du système d'analyse dans la région d'un plan d'applanation (15), ledit diamètre d₂ correspondant à une distance entre deux points opposés d'un plan de section longitudinale de la cornée dans la direction de l'axe visuel, chacun desdits points représentant les points de la cornée les plus proches au système d'analyse dans le plan de section longitudinale, la pression intraoculaire étant dérivée en tenant compte des diamètres d₁ et d₂ de la cornée, la pression intraoculaire étant dérivée en comparant les valeurs relatives des diamètres en question avec des valeurs stockées dans une banque de données.

2. Procédé d'analyse selon la revendication 1,
**caractérisé en ce**
**qu'**une pression de pompe pour produire le jet d'air se déroule en forme d'une courbe en cloche (13) par rapport à une durée du jet d'air.

3. Procédé d'analyse selon la revendication 1 ou 2,
**caractérisé en ce**
**qu'**une pression de pompe maximale pour produire le jet d'air est la même pour des mesures précédentes et subséquentes.

4. Procédé d'analyse selon l'une quelconque des revendications précédentes,
**caractérisé en ce**
**qu'**une pression de pompe pour produire le jet d'air est mesurée quand un point d'applanation de la cornée (10) est atteint.

5. Procédé d'analyse selon l'une quelconque des revendications précédentes,
**caractérisé en ce**
**qu'**une vitesse de la cornée (10) mue est mesurée, ladite vitesse étant mesurée pour dériver la propriété de matériau.

6. Procédé d'analyse selon l'une quelconque des revendications précédentes,
**caractérisé en ce**
**qu'**une dimension d'une surface d'applanation (14, 18) plane est mesurée quand un point d'applanation de la cornée (10) est atteint, ladite dimension étant mesurée pour dériver la propriété de matériau.

7. Procédé d'analyse selon l'une quelconque des revendications précédentes,
**caractérisé en ce**
**qu'**un rapport entre le diamètre d₁ de la première surface d'applanation (14) de la cornée (10) et un diamètre d₃ d'une deuxième surface d'applanation de la cornée est déterminé, ledit rapport étant déterminé pour dériver la propriété de matériau.

8. Procédé d'analyse selon l'une quelconque des revendications précédentes,
**caractérisé en ce**
**qu'**une amplitude d'un déroulement géométrique de la déformation de la cornée (10) est dérivée des images en coupe de la cornée, ladite amplitude étant dérivée pour dériver la propriété de matériau.

9. Procédé d'analyse selon l'une quelconque des revendications précédentes,
**caractérisé en ce**
**qu'**une courbure de la cornée (10) non déformée et/ou déformée est dérivée des images en coupe de la cornée, ladite courbure étant dérivée pour dériver la propriété de matériau.

10. Procédé d'analyse selon l'une quelconque des revendications précédentes,
**caractérisé en ce que**
la déformation de la cornée (10) est continuée par une oscillation libre de la cornée et **en ce que** l'oscillation libre de la cornée est déterminée comme autre propriété de matériau.

11. Procédé d'analyse selon la revendication 10,
**caractérisé en ce**
**qu'**une fréquence et/ou amplitude de l'oscillation libre sont/est mesurée(s).

12. Procédé d'analyse selon l'une quelconque des revendications précédentes,
**caractérisé en ce que**
le système d'observation comprend une caméra et une unité d'illumination dans une configuration de Scheimpflug, les images en coupe étant acquises moyennant la caméra.

13. Système d'analyse ophtalmologique destiné à mesurer une pression intraoculaire dans un œil (11), comprenant une unité d'actionnement à l'aide de laquelle une cornée (10) de l'œil peut être déformée sans contact, ladite unité d'actionnement pouvant diriger un jet d'air sur l'œil pour déformer la cornée, un système d'observation à l'aide duquel la déformation de la cornée peut être observée et acquise, ledit système d'observation pouvant acquérir des images en coupe de la cornée non déformée et déformée, et une unité d'analyse à l'aide de laquelle la pression intraoculaire peut être dérivée des images en coupe de la cornée, ladite unité d'analyse étant configurée pour dériver une propriété du matériau de la cornée des images en coupe de la cornée,
**caractérisé en ce**
**qu'**une déformation maximale de la cornée est dérivée des images en coupe de la cornée, un diamètre d₁ d'une première surface d'applanation (14) de la cornée et un diamètre d₂ d'une surface de déformation (18) de la cornée différant de la première surface d'applanation étant dérivés comme propriété de matériau pour la déformation maximale de la cornée par rapport à l'apex (16) de la cornée, ladite première surface d'applanation étant orthogonale par rapport à un axe visuel (12) de l'œil ou à un axe de dispositif du système d'analyse dans la région d'un plan d'applanation (15), ledit diamètre d₂ correspondant à une distance entre deux points opposés d'un plan de section longitudinale de la cornée dans la direction de l'axe visuel, chacun desdits points représentant les points de la cornée les plus proches au système d'analyse dans le plan de section longitudinale, la pression intraoculaire étant dérivée en tenant compte des diamètres d₁ et d₂ de la cornée, la pression intraoculaire étant dérivable en comparant les valeurs relatives des diamètres en question avec des valeurs stockées dans une banque de données.
